(19) Europäisches Patentamt
European Patent Office
Office européen des brevets

(11) **EP 4 205 641 B1**

(12) **FASCICULE DE BREVET EUROPEEN**

(45) Date de publication et mention de la délivrance du brevet:
**20.08.2025  Bulletin 2025/34**

(21) Numéro de dépôt: **22216906.2**

(22) Date de dépôt: **28.12.2022**

(51) Classification Internationale des Brevets (IPC):
*A61B 5/00* $^{(2006.01)}$        *G01N 21/64* $^{(2006.01)}$
*G02B 21/16* $^{(2006.01)}$       *G02B 21/36* $^{(2006.01)}$
*G06T 11/00* $^{(2006.01)}$

(52) Classification Coopérative des Brevets (CPC):
**G01N 21/6456; A61B 5/0071; A61B 5/0073; G06T 11/003**

(54) **PROCÉDÉ D ESTIMATION D'UNE DISTRIBUTION SPATIALE TRIDIMENSIONNELLE DE FLUORESCENCE, À L INTÉRIEUR D'UN OBJET**

VERFAHREN ZUR SCHÄTZUNG EINER DREIDIMENSIONALEN RÄUMLICHEN FLUORESZENZVERTEILUNG IN EINEM OBJEKT

METHOD FOR ESTIMATING A THREE-DIMENSIONAL SPATIAL DISTRIBUTION OF FLUORESCENCE, INSIDE AN OBJECT

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité:  **29.12.2021  FR 2114627**

(43) Date de publication de la demande:
**05.07.2023  Bulletin 2023/27**

(73) Titulaire: **Commissariat à l'Energie Atomique et aux Energies Alternatives**
**75015 Paris (FR)**

(72) Inventeurs:
• **HERVE, Lionel**
  **38054 GRENOBLE cedex 09 (FR)**
• **ALLIER, Cédric**
  **38054 GRENOBLE cedex 09 (FR)**

(74) Mandataire: **INNOV-GROUP**
**209 Avenue Berthelot**
**69007 Lyon (FR)**

(56) Documents cités:
**EP-A2- 1 971 258        WO-A1-2011/038006**
**US-A1- 2011 090 316     US-A1- 2012 211 671**

**Description**

**DOMAINE TECHNIQUE**

Le domaine technique de l'invention est la localisation tridimensionnelle de zones fluorescentes dans un objet.

**ART ANTERIEUR**

L'imagerie de fluorescence est une technique permettant de localiser des marqueurs fluorescents dans un corps humain ou animal. Une des principales applications est la localisation de marqueurs fluorescents, ou fluorophores, ces derniers ciblant des cellules d'intérêt, par exemple des cellules cancéreuses. Le protocole consiste à injecter ces marqueurs dans l'organisme avant un examen par imagerie de fluorescence. Parce qu'elle permet d'acquérir une image indiquant la localisation des différentes zones cancéreuses, l'imagerie de fluorescence constitue un complément utile, voire une alternative, à l'utilisation de traceurs radioactifs.

WO2011038006 décrit un procédé de reconstruction de fluorescence, permettant de localiser des marqueurs fluorescents dans un échantillon, en prenant l'hypothèse d'une répartition homogène de l'indice de réfraction dans l'échantillon.

Dans le domaine de la microscopie, une difficulté dans la reconstruction de la fluorescence est la distribution spatiale de l'indice de réfraction de l'échantillon, qui peut ne pas être uniforme, en particulier lorsque l'échantillon est un tissu biologique réelle. En effet, les échantillons biologiques présentent des inhomogénéités d'indice de réfraction, par exemple au niveau des parois ou des autres structures cellulaires (membrane lipidique, noyau). Dans les milieux de cultures, de telles inhomogénéités apparaissent à l'interface entre les cellules et le milieu de culture.

Une solution, visant à limiter l'influence de la non-uniformité des indices de réfraction, consiste à utiliser un agent clarifiant. Ce dernier peut être un solvant détruisant des membranes. Un tel procédé permet d'obtenir des images de fluorescences en 3D qui ne souffrent pas de la non-uniformité de l'indice de réfraction. Cependant, le recours à un agent clarifiant est invasif et de fait ne permet pas un suivi, dans le temps, d'un échantillon.

L'inventeur propose une méthode de reconstruction de la distribution spatiale de fluorescence dans un échantillon, ne nécessitant pas le recours à un agent clarifiant. Il s'agit d'un procédé non invasif, n'affectant pas l'intégrité de l'échantillon. Le procédé décrit ci-après permet de prendre en compte une distribution spatiale non homogène d'indices de réfraction mesurée, cette dernière étant supposée connue. Elle peut par exemple être préalablement obtenue en mettant en œuvre une tomographie optique de diffraction.

**EXPOSE DE L'INVENTION**

Un premier objet de l'invention est un procédé de reconstruction d'une distribution spatiale de fluorescence, tridimensionnelle, à l'intérieur d'un objet, l'objet étant susceptible d'émettre une lumière de fluorescence sous l'effet d'une illumination dans une bande spectrale d'excitation, l'objet étant discrétisé en voxels objet, de telle sorte que :

- chaque voxel objet est susceptible d'émettre une lumière dans une bande spectrale de fluorescence ;
- la distribution spatiale de fluorescence définit une intensité d'émission de lumière de fluorescence dans chaque voxel objet;

le procédé comportant les étapes suivantes ,

- a) disposition de l'objet face à un capteur d'image, le capteur d'image étant configuré pour former une image dans un plan focal objet, le plan focal objet étant configuré pour s'étendre successivement en différentes profondeurs dans l'objet ;
- b) illumination de l'objet dans la bande spectrale d'excitation, et acquisition successive de plusieurs images élémentaires, le plan focal objet s'étendant respectivement aux différentes profondeurs de l'objet, chaque image étant définie selon des pixels, à chaque pixel correspondant une valeur d'intensité mesurée, les images élémentaires formant une image acquise tridimensionnelle, chaque pixel d'une image élémentaire formant un voxel image de l'image acquise ;
- c) initialisation de la distribution spatiale de fluorescence ;
- d) prise en compte de la distribution de fluorescence initiale ou résultant d'une itération précédente ;
- e) assignation d'une valeur aléatoire de phase à chaque voxel objet, et mise en œuvre d'un algorithme de propagation de la lumière à travers l'objet, de façon à estimer une amplitude complexe de l'onde lumineuse détectée par chaque voxel image;
- f) répétition de W fois de l'étape e), W étant un entier positif, de façon à obtenir, en chaque voxel image, W estimations d'amplitudes complexes ;

- g) en chaque voxel image, à partir des W estimations résultant de l'étape f), calcul d'une intensité reconstruite, l'ensemble des intensités reconstruites pour chaque voxel image formant une image reconstruite ;
- h) formation éventuelle d'une image différentielle, l'image différentielle correspondant à une comparaison entre l'image reconstruite et l'image acquise ;
- i) assignation d'une valeur aléatoire de phase à chaque voxel image de l'image acquise résultant de l'étape g) ou de l'image différentielle résultant de l'étape h), et mise en œuvre d'un algorithme de rétropropagation de la lumière à travers l'objet, de façon à obtenir, dans chaque voxel objet :

  • une amplitude complexe d'émission de fluorescence correspondant à l'image reconstruite résultant de l'étape g) ;
  • ou une amplitude complexe différentielle d'émission de fluorescence correspondant à l'image différentielle résultant de l'étape h) ;

- j) répétition de W' fois de l'étape i), W' étant un entier positif, de façon à obtenir, pour chaque voxel objet, W' estimations d'amplitudes complexes correspondant à l'image reconstruite ou W' estimations d'amplitudes complexes différentielles correspondant à l'image différentielle ;
- k) à partir des W' estimations résultant de l'étape j), calcul, en chaque voxel objet, d'une intensité d'émission ou d'une intensité d'émission différentielle ;
- l) mise à jour de la distribution spatiale de fluorescence dans l'objet à partir de l'intensité d'émission ou de l'intensité d'émission différentielle calculée dans chaque voxel de l'objet lors de l'étape k) ;
- m) réitération des étapes d) à l) jusqu'à l'atteinte d'un critère d'arrêt des itérations.

Selon un mode de réalisation, le procédé comporte, à chaque itération, les étapes suivantes :

• i-bis) assignation d'une valeur aléatoire de phase à chaque voxel image de l'image acquise lors de b) et mise en œuvre d'un algorithme de rétropropagation de la lumière à travers l'objet, de façon à estimer, dans chaque voxel objet, une amplitude complexe d'émission de fluorescence correspondant à l'image acquise ;
• j-bis) répétition de W" fois de l'étape i-bis), W" étant un entier positif, de façon à obtenir, pour chaque voxel objet, W" estimations d'amplitudes complexe d'émission de fluorescence correspondant à l'image acquise ;
• k-bis) à partir des W" estimations résultant de l'étape j-bis), calcul d'une intensité d'émission, en chaque voxel objet, correspondant à l'image acquise;

le procédé étant tel que lors de l'étape l), la mise à jour est effectuée à partir d'un ratio calculé pour chaque voxel objet, entre l'intensité d'émission correspondant à l'image acquise et l'intensité d'émission correspondant à l'image reconstruite.

Lors de chaque répétition de l'étape i-bis), la valeur de phase assignée à chaque voxel image de l'image acquise peut correspond à la valeur de phase assignée au même voxel image de l'image reconstruite lors d'une étape i) de la même itération.

Lors de chaque étape k-bis), pour chaque voxel objet, l'intensité d'émission peut être calculée à partir d'une moyenne des W" intensités d'émission estimées, pour ledit voxel objet, lors de chaque étape i-bis).

Selon un mode de réalisation,

- lors de l'étape h), l'image différentielle est une différence quadratique entre l'image reconstruite et l'image acquise ;
- chaque étape i) comporte une obtention, dans chaque voxel image, d'une amplitude complexe différentielle, calculée à partir de l'image différentielle ;
- l'étape k) comporte un calcul d'une intensité d'émission différentielle, en chaque voxel objet, correspondant à l'image différentielle calculée en h) ;
- lors de l'étape l), la mise à jour de la distribution spatiale de fluorescence dans l'objet est effectuée de manière à minimiser l'intensité d'émission différentielle des voxels objets.

De préférence,

- lors de chaque étape g), pour chaque voxel image, l'intensité reconstruite est calculée à partir d'une moyenne du carré des modules des W estimations d'amplitudes complexes, pour ledit voxel image, lors de chaque étape e) ;
- lors de chaque étape k), pour chaque voxel objet, l'intensité d'émission est calculée à partir d'une moyenne du carré des modules des W' estimations amplitudes complexes d'émission de fluorescence, pour ledit voxel objet, lors de chaque étape i).

Selon une possibilité :

- lors de chaque étape e), une longueur d'onde est assignée à chaque voxel objet, dans la bande spectrale de fluorescence, la longueur d'onde étant assignée de façon aléatoire ou selon une distribution de probabilité prédéterminée ;
- lors de chaque étape i), une longueur d'onde est assignée à chaque voxel image, dans la bande spectrale de fluorescence, la longueur d'onde étant assignée de façon aléatoire ou selon une distribution de probabilité prédéterminée.

Lors de l'étape e) et lors de l'étape i), l'algorithme de propagation de la lumière peut mettre en œuvre un algorithme de propagation unidirectionnel, de type méthode de propagation de faisceau, de façon à déterminer une lumière se propageant à travers un voxel à partir de la lumière se propageant à partir d'un voxel adjacent. L'indice de réfraction de l'objet peut varier entre deux voxels objet différents. L'algorithme de propagation de la lumière prend alors en compte une variation de l'indice de réfraction entre deux voxels objet adjacents.

Un deuxième objet de l'invention est un dispositif d'observation d'un objet, le dispositif comportant :

- un capteur d'image, configuré pour former une image dans un plan objet, la distance entre le capteur d'image et le plan objet étant variable ;
- une source de lumière, configurée pour illuminer un objet ;
- une unité de traitement, configurée pour recevoir des images acquises par le capteur d'image, et mettre en œuvre les étapes c) à l) d'un procédé selon le premier objet de l'invention.

Un troisième objet de l'invention est un support d'enregistrement, lisible par un ordinateur ou pouvant être connecté à un ordinateur, le support d'enregistrement étant configuré pour mettre en œuvre les étapes c) à m) d'un procédé selon le premier objet de l'invention à partir d'images formées par un capteur d'image en différentes profondeurs dans un objet..

L'invention sera mieux comprise à la lecture de l'exposé des exemples de réalisation présentés, dans la suite de la description, en lien avec les figures listées ci-dessous.

**FIGURES**

La figure 1A représente un exemple de dispositif permettant une mise en œuvre de l'invention.

La figure 1B montre un autre exemple de dispositif permettant une mise en œuvre de l'invention.

La figure 2 schématise les principales étapes d'un procédé selon l'invention.

La figure 3 illustre l'acquisition d'images en profondeur, dans l'objet, et la formation d'une image tridimensionnelle.

La figure 4 schématise un algorithme de propagation de faisceau à travers l'objet.

La figure 5A représente une coupe d'un objet utilisé pour effectuer des essais expérimentaux.

La figure 5B montre un résultat de l'application de l'algorithme de propagation de faisceau à travers l'objet.

La figure 5C montre une coupe d'une image tridimensionnelle de l'objet.

La figure 5D représente une distribution spatiale de fluorescence dans l'objet, résultant de la mise en œuvre du procédé décrit en lien avec la figure 2.

La figure 5E montre l'évolution d'un indicateur d'attache aux données en fonction des itérations de l'algorithme.

La figure 5F montre l'évolution de l'intensité de fluorescence de la distribution de fluorescence, cumulée selon la profondeur de l'objet, au cours des itérations successives.

**EXPOSE DE MODES DE REALISATION PARTICULIERS**

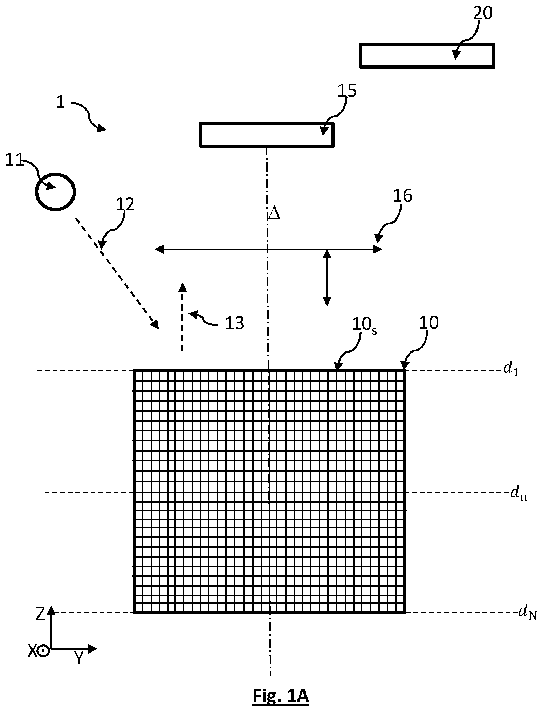

La figure 1A représente un dispositif permettant une mise en œuvre de l'invention. Le dispositif comporte une source de lumière 11, configurée pour illuminer un objet 10. L'objet est un volume solide prenant la forme d'un gel, que l'on souhaite analyser. Sous l'effet de l'illumination par la source de lumière, l'objet émet une lumière d'émission. La source de lumière émet une lumière d'illumination 12 dans un bande spectrale d'illumination. Une partie de la lumière émise par la source de lumière se propage à travers l'objet. Sous l'effet de l'illumination, l'objet émet une lumière de fluorescence 13.

La source de lumière peut être une source laser ou une diode électroluminescente. La source de lumière peut être fibrée, la lumière émise par la source de lumière étant guidée vers l'objet avec une fibre optique.

L'objet est susceptible de comporter des fluorophores, émettant la lumière de fluorescence, dans une bande spectrale de fluorescence, lorsqu'ils sont illuminés dans une bande spectrale d'excitation. Lorsque la bande spectrale d'illumination de la source de lumière est comprise dans la bande spectrale d'excitation, l'objet émet la lumière de fluorescence dans une bande spectrale de fluorescence. L'analyse de l'objet vise à déterminer la position des fluorophores dans l'objet. Pour cela, on cherche à déterminer une distribution spatiale tridimensionnelle d'émission de la lumière de fluorescence à l'intérieur de l'objet, de façon à pouvoir identifier les zones de l'objet présentant une concentration élevée de fluorophores.

L'objet est par exemple un tissu biologique, que l'on souhaite analyser afin d'identifier d'éventuelles singularités. L'objectif est d'identifier des concentrations locales de fluorophores dans l'objet, ces dernières pouvant être utilisés pour aider à la détermination d'un état pathologique.

L'objet 10 est discrétisé en voxels, dits « voxels objet ». Chaque voxel objet correspond à un volume élémentaire de l'objet. Il peut par exemple s'agir d'un volume de 100 nmx100 nm à 10 $\mu$m x 10 $\mu$m. Au niveau de chaque voxel objet, l'objet a un indice de réfraction, dans la bande spectrale de fluorescence. L'indice de réfraction peut être inhomogène, et varier entre un voxel objet et un autre voxel objet. La non-homogénéité de l'indice de réfraction est courante lorsque l'objet est un échantillon biologique. Dans la suite, chaque voxel objet est désigné par une coordonnée tridimensionnelle r.

Le dispositif comporte un capteur d'image pixelisé 15, configuré pour former une image de l'objet. Le capteur d'image 15 est couplé à un système optique 16, ce dernier permettant de conjuguer un plan objet, dit plan focal, avec le capteur d'image. Le capteur d'image et le système optique sont alignés selon un axe optique $\Delta$. L'ensemble formé par le capteur d'image et le système optique est configuré de façon que le plan focal puisse être translaté parallèlement à l'axe optique $\Delta$. Le système optique peut combiner un objectif et une lentille de tube. Le capteur d'image peut être un capteur de type CMOS. Le plan focal peut ainsi être translaté selon différentes profondeurs à l'intérieur de l'objet.

De façon alternative, le capteur d'image est associé à un diaphragme confocal, ce dernier permettant l'observation successive de différentes tranches de l'objet.

Ainsi, d'une façon générale, le capteur d'image est configuré pour acquérir des images dans différents plans, s'étendant à différentes profondeurs dans l'objet. L'objet est délimité par une surface $10_s$, formant une interface entre l'objet et un milieu ambiant dans lequel s'étend le capteur d'image. Le milieu ambiant est généralement de l'air. Une profondeur dans l'objet correspond à une distance par rapport à la surface $10_s$, parallèlement à l'axe optique $\Delta$. Le capteur d'image peut être couplé à un filtre spectral, par exemple de type passe-bande, de façon à détecter une onde lumineuse dans la bande spectrale d'émission.

Le dispositif comporte une unité de traitement 20, agencée pour traiter les images formées par le capteur d'image, de façon à estimer la distribution spatiale, tridimensionnelle, de d'émission de lumière de fluorescence dans l'objet.

La source de lumière est configurée pour émettre une lumière d'illumination 12, se propageant à travers l'objet. La lumière d'illumination s'étend dans un bande spectrale d'illumination à laquelle l'objet présente une transmittance non nulle. La bande spectrale d'illumination correspond ou est comprise dans la bande spectrale d'excitation des fluorophores susceptibles d'être présents dans l'objet.

Une difficulté est que la fluorescence est un phénomène non cohérent. Sous l'effet d'une illumination dans la bande spectrale d'excitation, deux fluorophores différents émettent généralement des ondes lumineuses non cohérentes, formant la lumière de fluorescence. Et cela même si la lumière d'excitation est cohérente.

Une autre difficulté est la non homogénéité de l'indice de réfraction optique de l'objet. Dans les méthodes de l'art antérieur, la distribution spatiale d'émission de fluorescence est obtenue par l'inversion d'une équation de type :

$$I = PSF \times F \quad (1)$$

où

- $F$ correspond à un vecteur dont chaque terme I(r) est l'intensité de fluorescence dans un voxel objet de coordonnée tridimensionnelle r: cela correspond à ce que l'on cherche à estimer.
- $I$ est une image de fluorescence formée par le capteur d'image : cela correspond à une mesure ;
- $PSF$ (Point Spread Function -Réponse impulsionnelle)est une matrice représentative de la réponse de l'instrument : cette matrice est établie par modélisation et peut être recalée par des essais expérimentaux.

Une telle formule est valide si l'indice optique est homogène ou peu hétérogène. L'hypothèse d'homogénéité de l'indice optique est acceptable lorsque l'épaisseur de l'objet est faible, ou lorsque l'objet a été préalablement traité par un agent de clarification, permettant une certaine homogénéisation de l'indice optique à l'intérieur de l'objet. L'agent de clarification est usuellement un solvant, détruisant les membranes lipidiques voire les noyaux des cellules. Cela permet de réduire des effets de sauts d'indice à l'intérieur de l'objet.

La propagation de la lumière dans un milieu, entre un voxel objet r de l'objet et un pixel r' du capteur d'image, est modélisée par :

$$U^\lambda(r') = \int G^\lambda(r', r) s^\lambda(r) dr \quad (2)$$

où :

- $U^\lambda(r')$ est l'amplitude complexe de l'onde lumineuse au pixel image r'. L'amplitude complexe comporte une partie réelle et une partie imaginaire, la partie imaginaire correspondant au déphasage.
- $s^\lambda(r)$ correspond à une amplitude complexe d'une onde lumineuse, en l'occurrence une onde lumineuse de fluorescence émise par le voxel objet $r$ de l'objet à la longueur d'onde $\lambda$.
- $G^\lambda(r', r)$ est une fonction complexe, permettant d'estimer l'amplitude complexe dans le pixel $r'$ à partir de chaque voxel objet $r$.

La fonction complexe $G^\lambda(r, r')$ décrit le transport de la lumière entre $r$ et $r'$. Elle est obtenue par résolution de l'équation de Helmholtz :

$$\Delta U^\lambda(r') + 4\pi^2 \frac{n^2}{\lambda^2} U^\lambda(r') = -s(r) \quad (3)$$

où $\Delta$ est l'opérateur Laplacien et $n$ est l'indice de réfraction complexe.

L'expression (3) décrit la propagation d'une onde lumineuse cohérente, monochromatique. Elle est mise en œuvre dans des procédés de reconstruction de l'art antérieur.

L'intensité mesurée par le pixel d'un capteur d'image situé à la coordonnée r', est telle que :

$$I(r') = Re \int E[U^\lambda(r') U^{\lambda*}(r')] d\lambda \quad (4)$$

où $E$ désigne l'opérateur espérance mathématique et * désigne l'opérateur complexe conjugué. L'intégration selon la longueur d'onde $\lambda$ permet une prise en compte d'une certaine largeur spectrale de l'émission d'une lumière dans chaque voxel objet $r$. Généralement, l'émission lumineuse n'est pas monochromatique et s'étend dans une bande spectrale d'émission, décrite par une distribution probabiliste spectrale P. Chaque terme $P^\lambda$ de la distribution spectrale P est une probabilité d'émission à la longueur d'onde $\lambda$. La distribution spectrale P est par exemple gaussienne.

L'utilisation de l'opérateur espérance mathématique traduit le fait que l'intensité est mesurée durant une durée longue par rapport à la fréquence de l'onde lumineuse atteignant le pixel. L'intensité mesurée correspond à une moyenne du module de l'amplitude complexe durant la durée de la mesure, c'est-à-dire la durée de l'acquisition de l'image par le capteur d'image.

En combinant (4) et (2), on obtient :

$$I(r') = Re \iiint G^\lambda(r', r_1) G^{\lambda*}(r', r_2) E[s^\lambda(r_1) s^{\lambda*}(r_2)] d\lambda dr_1 dr_2 \quad (5)$$

où $r_1$ et $r_2$ désignent des voxels objet.

La notation $s^{\lambda*}(r_2)$ correspond au complexe conjugué de $s^\lambda(r_2)$.

Compte tenu du caractère incohérent de l'émission de fluorescence, le terme $E[s^\lambda(r_1) s^{\lambda*}(r_2)]$ est nul sauf lorsque $r_1 = r_2$. Ainsi, $E[s^\lambda(r_1) s^{\lambda*}(r_2)]$ peut s'écrire :

$$E[s^\lambda(r_1) s^{\lambda*}(r_2)] = F(r_1) P^\lambda \delta(r_2 - r_1) \quad (6)$$

- $P^\lambda$ est la probabilité d'émission à la longueur d'onde $\lambda$ préalablement décrite ;

- $F(r_1)$ est une intensité d'émission de fluorescence dans chaque voxel objet $r_1$. La distribution $F$ correspond à la distribution tridimensionnelle d'émission de fluorescence dans l'objet, que l'on cherche à estimer ;
- $\delta$ correspond à une distribution de Dirac.

La combinaison de (5) et (6) donne :

$$I(r') = \iint P^\lambda \left| G^\lambda(r', r) \right|^2 F(r) d\lambda dr \quad (7)$$

L'expression (7) correspond à un modèle direct, reliant la distribution d'intensités d'émission $F$ à l'intensité mesurée au pixel $r'$. Elle peut être exprimée selon une forme matricielle :

$$I = L\,F \quad (7')$$

où

- $I$ est un vecteur dont la dimension correspond au nombre de pixels dans l'image, et dont chaque terme est une intensité de lumière de fluorescence émise par le voxel objet ;
- $L$ est une matrice de passage, dont chaque terme est $P^\lambda |G^\lambda(r', r)|^2$;
- $F$ est un vecteur dont la dimension correspond au nombre de voxels, et dont chaque terme est une intensité de lumière de fluorescence émise par le voxel objet ;

Cependant, l'objet analysé peut être discrétisé selon un nombre élevé de voxels objets, par exemple quelques millions, voire dizaines ou centaines de millions. De même, le nombre de points de mesure $r'$ peut être du même ordre de grandeur, notamment si l'on forme différentes images de l'objet, comme décrit par la suite. De ce fait, on ne peut pas établir une matrice de passage $L$, à chaque longueur d'onde, dont chaque terme serait égal à $P^\lambda |G^\lambda(r', r)|^2$. Cela serait trop consommateur de ressources. Par exemple si on considère un nombre de voxels objet r et un nombre de points de mesure $r'$ égaux à $100\,10^6$, une matrice de passage, pour chaque longueur d'onde, établie d'après le modèle direct aurait une taille de $100\,10^6 \times 100\,10^6$, ce qui est difficilement concevable.

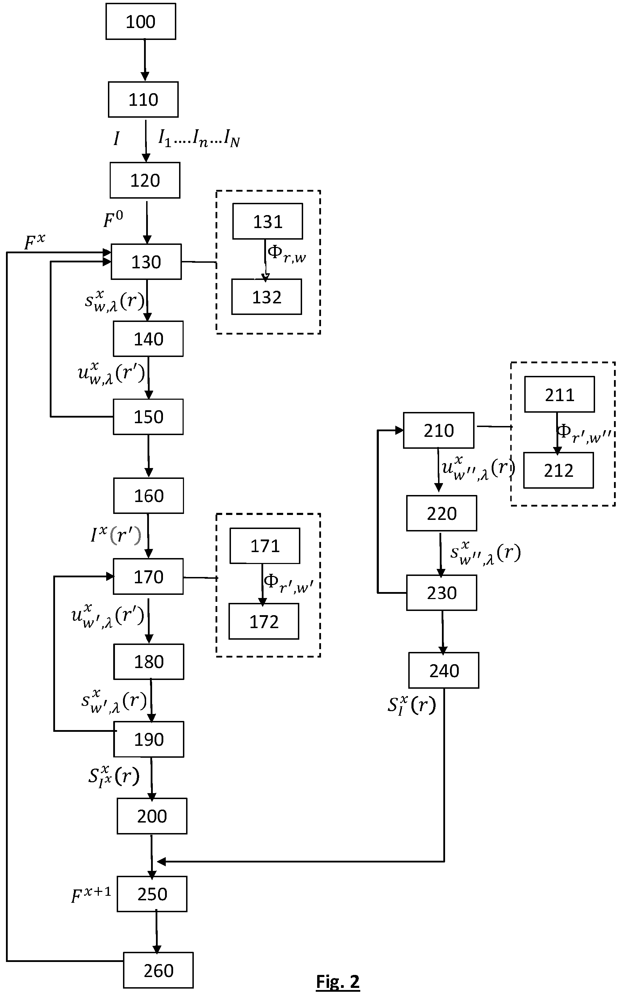

L'unité de traitement 20 est configurée pour mettre en œuvre un procédé de reconstruction, dont les principales étapes sont schématisées sur la figure 2. L'objectif est d'estimer, par itérations, un vecteur $F$, dont chaque terme $F(r)$ correspond à une intensité d'émission d'une onde lumineuse fluorescente dans un voxel objet $r$. $F$ est une carte de fluorescence de l'objet. La dimension de $F$ correspond au nombre de voxels objet.

Le procédé comporte tout d'abord des étapes permettant l'obtention de données mesurées.

Etape 100 : illumination de l'objet.

Au cours de l'étape 100, l'objet est illuminé par la source de lumière 11 dans une bande spectrale d'illumination. Dans l'exemple décrit, la bande spectrale d'illumination est une bande spectrale d'excitation d'un fluorophore potentiellement présent dans l'objet.

L'objet peut être interposé entre la source de lumière 11 et le capteur d'images 15, ce qui correspond à une configuration d'acquisition usuellement désignée par le terme « en transmission ». De façon alternative, comme représenté sur la figure 1A, l'acquisition peut être effectuée en réflexion, ou rétrodiffusion, la source de lumière 11 et le capteur d'image 15 étant disposés face à l'objet.

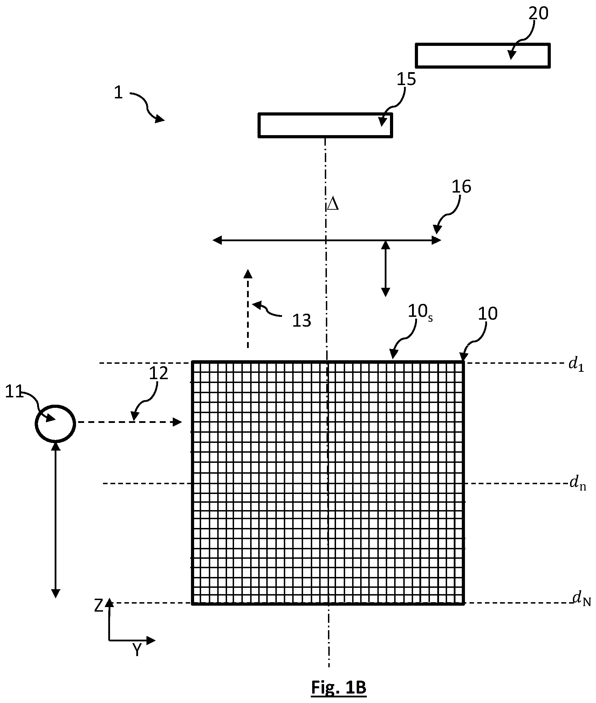

Selon une autre possibilité, la source de lumière 11 peut être mobile par rapport à l'objet au cours de l'acquisition. Par exemple, comme représenté sur la figure 1B, la source de lumière 11 peut générer un faisceau de lumière 12 formant une lame lumineuse étroite, perpendiculaire à l'axe optique. La source de lumière peut alors être translatée parallèlement à l'objet, de façon à illuminer successivement différentes couches de l'objet, chaque couche s'étendant de préférence perpendiculairement à l'axe optique du capteur d'image.

Etape 110 : Acquisition d'images de l'objet

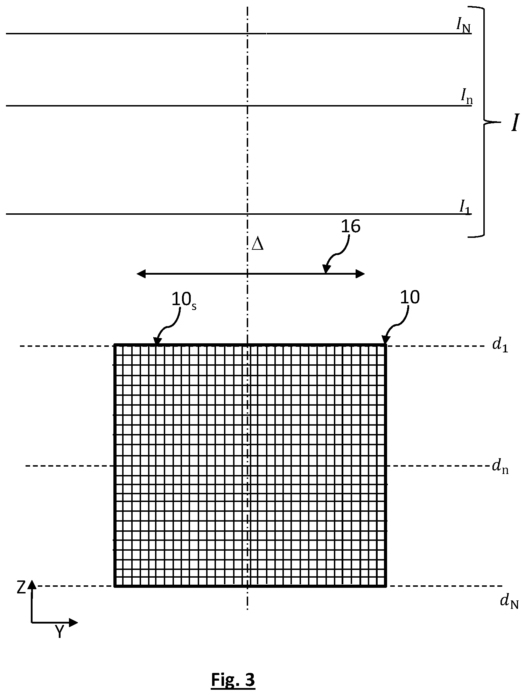

Au cours de cette étape, on acquiert une ou plusieurs images élémentaires $I_1....I_n...I_N$ de l'objet, en différentes profondeurs. La figure 3 schématise une configuration d'acquisition selon laquelle le plan focal du capteur d'image est successivement déplacé en différentes profondeurs dans l'objet, ce qui correspond à un mode de réalisation préféré. La source de lumière reste fixe. Le capteur d'image acquiert autant d'images que de distances focales, chaque distance focale étant associée à une profondeur $dn$ dans l'objet. On obtient ainsi des images élémentaires $I_1....I_n...I_N$, $N$ correspondant au nombre d'images élémentaires. Les images élémentaires $I_1....I_n...I_N$ sont respectivement associées aux profondeurs $d_1....d_n...d_N$. Dans la suite, les images élémentaires sont combinées pour former une image acquise notée $I$ : il s'agit d'une image tridimensionnelle de l'objet. L'image acquise est discrétisée en voxels, dits voxels image $I(r)$, chaque voxel image étant un pixel d'une image élémentaire $I_n$ formant l'image acquise $I$.

De façon alternative, le capteur d'image a une distance focale fixe et il est déplacé par rapport à l'objet, de façon que le plan focal soit successivement déplacé en différentes profondeurs $d_1....d_n...d_N$ dans l'objet.

De façon alternative, le capteur d'image reste fixe et on utilise une source de lumière émettant la lumière selon une lame étroite de lumière. La source de lumière est ensuite translatée par rapport à l'objet, de préférence parallèlement à l'axe optique. Ainsi, au cours de chaque illumination, on éclaire une couche de l'objet parallèle au plan focal du capteur d'image, dont l'épaisseur est inférieure à l'écart entre deux profondeurs successives $d_n$, $d_{n-1}$. Le capteur acquiert une image en chaque position de la source de lumière. On forme ainsi successivement différentes images correspondant respectivement à différentes profondeurs de l'objet.

D'une façon générale, l'étape 110 vise à obtenir différentes images $I_1....I_n...I_N$ représentatives de différentes profondeurs de l'objet $d_1....d_n...d_N$. Le fait d'acquérir des images en disposant de plans focaux à différentes profondeurs permet d'obtenir des images dans lesquels les sources fluorescentes, dans l'objet, apparaissent de façon plus nettes. Cela permet d'obtenir une reconstruction plus précise.

Les étapes qui suivent sont mises en œuvre par l'unité de traitement 20. Cette dernière comporte un microprocesseur relié à une mémoire. La mémoire comporte des instructions permettant la mise en œuvre du traitement décrit par la suite. La mémoire de l'unité de traitement comporte l'image $I$ acquise lors de l'étape 110.

Etape 120 : Initialisation

Au cours de cette étape, la distribution spatiale de fluorescence dans l'objet est initialisée. Chaque terme $F(r)$ prend une valeur initiale $F^0(r)$ déterminée aléatoirement ou prédéfinie.

Les étapes 130 à 250 décrites par la suite sont effectuées de façon itérative.

Les étapes 130 à 150 vise à estimer, en chaque voxel image $r'$, une intensité reconstruite $I^x(r')$ en chaque voxel image $r'$ de l'image acquise $I$. $x$ est un entier correspondant à chaque rang d'itération. Compte tenu du fait qu'il n'est pas possible de recourir à un modèle direct explicite, tel qu'explicité dans (7), chaque intensité reconstruite $I^x(r')$ est estimée par une approche statistique.

Etape 130 : définition d'amplitudes complexes d'émission dans chaque voxel objet à partir de la distribution d'émission initiale $F^0$ ou d'une distribution d'émission résultant d'une itération précédente $F^x$.

Au cours de cette étape, le rang de l'itération est incrémenté. On dispose d'une distribution de fluorescence $F^x$ résultant soit de l'initialisation, soit d'une itération précédente. L'objectif des itérations est d'effectuer un mise à jour de la distribution de fluorescence de façon à obtenir, suite à chaque itération $x$, une distribution de fluorescence $F^x$. La distribution de fluorescence $F^x$ est définie en chaque voxel objet r. Chaque terme $F^x(r)$ de la distribution de fluorescence correspond à une intensité de fluorescence dans le voxel objet r.

Sous-étape 131 : Assignation de valeurs de phase à chaque voxel objet.

Comme précédemment indiqué, l'émission de fluorescence est un phénomène incohérent. Deux sources de lumière fluorescentes ponctuelles respectivement disposées dans deux voxels objet différents émettent respectivement deux ondes lumineuses de fluorescence non cohérentes, et cela même si la lumière d'excitation est une onde lumineuse cohérente. Afin de tenir compte de la non cohérence des émissions par les voxels objets, une valeur de phase aléatoire $\Phi_{r,w}$ est assignée à chaque voxel objet $r$. $w$ est un rang de répétition décrit par la suite.

Sous-étape 132 : Assignation d'une longueur d'onde

Au cours de cette étape, on assigne une longueur d'onde de fluorescence $\lambda$ à chaque voxel objet. De préférence, la longueur d'onde de fluorescence $\lambda$ est assignée en prenant en compte la probabilité d'émission $P^\lambda$ dans la bande spectrale d'émission, définie par la distribution de probabilités $P$, cette dernière étant, dans cet exemple, considérée comme gaussienne.

La sous-étape 132 est optionnelle. Elle est mise en œuvre si l'on souhaite tenir compte d'une certaine largeur de la bande spectrale d'émission, ce qui est plus réaliste.

Les sous-étapes 131 et 132 permettent de définir, en chaque voxel objet, une amplitude complexe d'émission de fluorescence $s_{w,\lambda}^x(r)$, en fonction de $F^x$, telle que :

$$s_{w,\lambda}^x(r) = \sqrt{F^x(r)}e^{i\Phi_{r,w}} \quad (8)$$

L'amplitude complexe d'émission de fluorescence correspond à l'amplitude complexe d'une onde lumineuse de fluorescence émise dans le voxel objet $r$.

Etape 140: Propagation

Au cours de cette étape, on applique un algorithme de propagation de la lumière permettant, de modéliser une amplitude complexe d'émission de fluorescence $s_{w,\lambda}^x(r)$, correspondant à une source de fluorescence localisée dans le voxel objet $r$. L'onde lumineuse émise au niveau de chaque voxel objet $r$ se propage à travers l'objet 10, vers le capteur d'image 15.

L'algorithme de propagation est basé sur une propagation d'une onde lumineuse cohérente, selon les expressions (2) et (3). Un aspect important de l'invention est que la prise en compte du caractère incohérent des émissions dans chaque voxel objet est effectuée lors de la sous-étape 131 au cours de laquelle une phase aléatoire est assignée à chaque voxel objet. De même, la prise en compte du caractère non monochromatique de l'émission de lumière, dans chaque voxel, est réalisée dans la sous-étape 132.

Au niveau de chaque voxel image, on peut estimer une amplitude complexe telle que :

$$u_{w,\lambda}^{x}(r') = \int G^{\lambda}(r',r) s_{w,\lambda}^{x}(r) dr \quad (9)$$

En tenant compte de la discrétisation de l'objet en voxels objet, cette expression peut être écrite sous forme matricielle :

$$U = G S_{w}^{x} \quad (9')$$

où

- $U$ est un vecteur dont chaque terme est $u_{w,\lambda}^{x}(r')$ ;

- $S_{w}^{x}$ est un vecteur dont chaque terme est $s_{w,\lambda}^{x}(r)$

- $G$ est une matrice de passage dont chaque terme est égal à $G^{\lambda}(r', r)$

L'expression (9') ne peut pas être déterminée analytiquement, compte tenu de la taille trop importante que prendrait la matrice $G$.

La valeur de $u_{w,\lambda}^{x}(r')$, en chaque voxel image, est approchée en mettant en œuvre un algorithme de propagation de type méthode de propagation de faisceau, usuellement désignée par l'acronyme BPM signifiant Beam Propagation Method. Il s'agit d'un algorithme connu de l'homme du métier, permettant de simuler la propagation de la lumière selon un modèle de propagation unidirectionnel. L'algorithme de propagation peut être appliqué de proche en proche, de façon à modéliser, dans chaque voxel objet, une amplitude complexe entrante et une amplitude complexe sortante de l'onde lumineuse de fluorescence se propageant à travers le voxel.

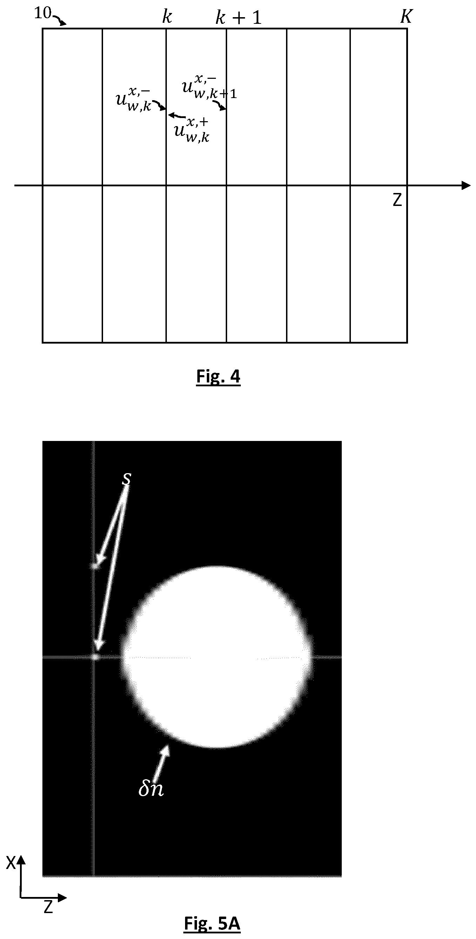

La figure 4 schématise une mise en œuvre d'un tel algorithme. Les voxels objet sont répartis par couches, à chaque couche étant assignée un indice $k$. $k = 1$ correspond à la couche la plus profonde. L'algorithme permet de modéliser une propagation de l'amplitude complexe entre les voxels situés à une profondeur $k + 1$ de l'objet vers les voxels situés à une profondeur $k$ de l'objet.

Si :

- $u_{w,k}^{x,-}(r)$ désigne l'amplitude complexe de l'onde lumineuse incidente à un voxel objet $r$ de la couche $k$ ;

- si $u_{w,k}^{x,+}(r)$ désigne l'amplitude complexe de l'onde lumineuse se propageant du voxel objet r de la couche $k$ vers un voxel adjacent de la couche $k + 1$, selon une direction de propagation ;

en l'absence d'émission de lumière de fluorescence dans chaque voxel de la couche $k$, $u_{w,k}^{x,+}(r)$ est telle que :

$$u_{w,k}^{x,+}(r) = u_{w,k}^{x,-}(r). e^{\frac{2i\pi.\delta n_k(r).\Delta z}{\lambda}} \quad (10)$$

et

$$\delta n_k(r) = n_k(r) - n_0 \quad (10')$$

$n_0$ est un indice moyen de l'objet : il est soit mesuré soit prédéterminé. Par exemple, dans le cas d'un objet biologique, $n_0 = 1.33$.

$\Delta z$ correspond à l'épaisseur du voxel $r$.

La prise en compte du terme $n_k(r)$ montre que l'algorithme de propagation permet une prise en compte d'une variation d'indice de réfraction dans l'objet.

A partir de $u_{w,k}^{x,+}(r)$, obtenu dans (10), on peut estimer $u_{w,k+1}^{x,-}$, relatif au voxel de rang k+1 selon :

$$u_{w,k+1}^{x,-} = u_{w,k}^{x,+}(r) * H_{\Delta z}^{\lambda} \quad (11)$$

L'expression (11) peut être exprimée dans le domaine fréquentiel : $TF[u_{w,k+1}^{x,-}(r)] = TF[u_k^+(r)] \odot H_{\Delta z}^{\lambda}$ (11'), *TF* désignant l'opérateur transformée de Fourier, avec

$$H_{\Delta z}^{\lambda}(\mu) = \exp\left( i2\pi\Delta z \sqrt{\frac{n_0^2}{\lambda^2} - \mu^2} \right) \quad (12)$$

où $\mu$ correspond à une coordonnée r exprimée dans le domaine des fréquences spatiales.

Lorsque le voxel objet *r* comporte une source d'émission de fluorescence $s_{w,\lambda}^x(r)$, déterminée par la distribution de fluorescence *Fˣ*, l'expression (11') devient,

$$TF[u_{w,k}^{x,-}(r)] = TF[[u_{w,k}^{x,+}(r)] \odot H_{\Delta z}^{\lambda} + \frac{TF[s_{w,\lambda}^x(r)]}{C} \quad (13)$$

où $s_{w,\lambda}^x(r)$ est explicité dans (8) et

$$C = \sqrt{1 - \frac{\lambda^2 \mu^2}{n_0^2}} \quad (13')$$

On peut ainsi calculer une propagation de l'amplitude complexe ( $u_{w,k=1}^{x,-}(r) \rightarrow u_{w,k=1}^{x,+}(r) \rightarrow u_{w,k=2}^{x,-}(r) \rightarrow u_{w,k=2}^{x,+}(r) \rightarrow \cdots \rightarrow u_{w,k=K}^{x,-}(r) \rightarrow u_{w,k=K}^{x,+}(r)$ ) où *K* correspond au nombre total de couches considérées dans le modèle de propagation BPM. Lorsqu'on atteint la surface de l'objet $10_s$, l'onde lumineuse se propage librement jusqu'au système optique, puis du système optique jusqu'au capteur d'image.

Au cours de cette étape, on estime l'amplitude complexe $u_{w,\lambda}^x(r)$ de l'onde lumineuse de fluorescence se propageant, de proche en proche, à travers chaque voxel de l'objet. L'onde lumineuse $u_{w,k=K}^{x,+}(r)$ émanant de chaque voxel de la dernière couche de l'objet, est ensuite propagée jusqu'à l'optique, puis jusqu'au capteur d'image. Dans ce modèle, le champ optique émanant de l'objet résulte uniquement des sources de fluorescence $s_{w,\lambda}^x(r)$ disposées en chaque voxel de l'objet selon la distribution de fluorescence *Fˣ*. Le champ incident à l'objet, c'est-à-dire incident à la couche *k* = 1, peut être considéré comme nul.

Suite à l'étape 140, on dispose d'une estimation de l'amplitude complexe $u_{w,\lambda}^x(r')$ en chaque voxel image *r'*.

Etape 150 : répétition des étapes 130 et 140, *W* étant un entier désignant le nombre de répétitions. *W* est par exemple égal à 1000.

Suite à l'étape 150, on dispose, en chaque voxel image, de *W* valeurs d'amplitude complexe $u_{w,\lambda}^x(r')$. Chaque amplitude complexe $u_{w,\lambda}^x(r')$ modélise à une onde amplitude complexe de l'onde lumineuse détectée par le voxel image *r'* sachant la distribution de fluorescence *Fˣ* résultant de l'itération précédente et sachant les distributions de phase et de longueur d'onde prises en compte dans les étapes 131 et 132.

Etape 160 : Obtention d'une image reconstruite *Iˣ*

A partir *des W* valeurs d'amplitude complexe $u_{w,\lambda}^x(r')$, on estime, en chaque voxel image, une intensité dite intensité reconstruite *Iˣ(r)*. L'intensité reconstruite est obtenue à partir d'une moyenne des carrés des modules des *W* amplitudes complexes $u_{w,\lambda}^x(r')$ résultant de l'étape 150. Ainsi,

$$I^x(r') = |\bar{u}_\lambda^x(r')|^2 = \frac{1}{W}\sum_w |u_{w,\lambda}^x(r')|^2 \quad (14)$$

L'ensemble des valeurs $I^x(r')$ forme une image reconstruite $I^x$.

Les étapes 130 à 150 permettent d'obtenir, statistiquement, l'image reconstruite $I^x$ telle que

$$I^x \approx L\, F^x \quad (15).$$

Le symbole $\approx$ signifie « approchant ».

Dans (15), l'image reconstruite $I^x$ est exprimée de façon vectorielle, par concaténation des voxels image. De même, $F^x$ est exprimée sous forme vectorielle, par concaténation des voxels objet.

La matrice de passage $L$ n'est pas déterminée analytiquement. Elle est implicite, en étant approchée par les propagations successivement mises en œuvre dans l'étape 140 et dont les résultats sont moyennés lors de l'étape 160. Autrement dit, les étapes 140 à 160 sont équivalentes à la définition de la matrice de passage $L$. On a remplacé une détermination explicite de $L$ par un moyennage de $w$ répétitions des étapes 140 et 150.

<u>Etape 170</u> : Définition d'amplitudes complexes dans chaque voxel image de l'image reconstruite $I^x$.

Au cours de cette étape, on dispose, en chaque voxel image de l'image reconstruite, d'une intensité reconstruite. L'intensité reconstruite, en chaque voxel image, correspond à $I^x(r')$ résultant de l'étape 160.

<u>Sous-étape 171</u> : Assignation de valeurs de phase à chaque voxel image $r'$ de l'image reconstruite.

De façon analogue à l'étape 131, une valeur de phase aléatoire $\Phi_{r',w'}$ est assignée à chaque voxel image. $w'$ est un rang de répétition décrit par la suite.

<u>Sous-étape 172</u> : Assignation d'une longueur d'onde

De façon analogue à l'étape 132 on assigne une longueur d'onde d'émission à chaque voxel image. La longueur d'onde d'émission est assignée en prenant en compte la probabilité d'émission $P^\lambda$ dans la bande spectrale d'émission, définie par la distribution de probabilités $P$. De même que la sous-étape 132, la sous-étape 162 est optionnelle.

Les sous-étapes 171 et 172 permettent de définir, en chaque voxel image $r'$, une amplitude complexe $u_{w,\lambda}^x(r')$ telle que :

$$u_{w',\lambda}^x(r') = \sqrt{I^x(r')}\, e^{i\Phi_{r',w'}} \quad (16)$$

. Chaque amplitude complexe $u_{w,\lambda}^x(r')$ est calculée à partir de l'image reconstruite $I^x$ résultant de l'étape 160. Chaque amplitude complexe correspond à une amplitude d'une onde lumineuse détectée dans le voxel image $r'$.

<u>Etape 180</u>: Rétropropagation

Au cours de cette étape, on applique un algorithme de rétropropagation de la lumière permettant de modéliser une rétropropagation d'une onde lumineuse à partir du capteur d'image, vers chaque voxel objet $r$. Il s'agit de déterminer un ensemble de valeurs d'amplitudes complexe d'émission de fluorescence $s_{w',\lambda}^x(r)$, dans chaque voxel objet, expliquant chaque amplitude complexe résultant de l'étape 170.

L'algorithme de rétropropagation est analogue à celui mise en œuvre dans l'étape 140 : méthode de propagation de faisceau BPM. Il permet d'estimer $s_{w',\lambda}^x(r)$, telle que

$$s_{w',\lambda}^x(r) = \int G^{\lambda H}(r,r')u_{w',\lambda}^x(r')dr' \quad (17)$$

où $H$ désigne l'opérateur Hilbertien.

Suite à la rétropropagation, on dispose, en chaque voxel objet, d'une amplitude complexe d'émission de fluorescence $s_{w',\lambda}^x(r)$. Cela revient à effectuer, par modélisation, l'opération matricielle :

$$s_{w',\lambda}^x = G^{\lambda H}U^x \quad (18)$$

où :

- $s_{w',\lambda}^{x}$ est un vecteur dont chaque terme est égal à une amplitude estimée d'une onde lumineuse de fluorescence $s_{w',\lambda}^{x}(r)$ émise dans chaque voxel objet ; $s_{w',\lambda}^{x}$ est obtenu par rétropropagation de $I^{x}$.

- $U^{x}$ est un vecteur dont chaque terme est égal à une amplitude de fluorescence détectée dans chaque voxel image $u_{w',\lambda}^{x}(r')$, en se basant sur l'image reconstruite $I^{x}$;

- $G^{\lambda H}$ est une matrice de passage, dont chaque terme est $G^{\lambda H}(r, r')$.

La matrice de passage $G^{\lambda H}$ ne peut pas être explicitée, car elle serait de taille trop importante. L'algorithme de rétropropagation permet d'estimer $s_{w',\lambda}^{x}$ à partir de $U^{x}$.

Etape 190 : répétition des étapes 170 et 180, $W'$ étant un entier désignant le nombre de répétitions. $W'$ est par exemple égal à 1000.

Suite à l'étape 190, on dispose, en chaque voxel objet, de $W'$ valeurs d'amplitudes complexe d'émission $s_{w,\lambda}^{x}(r')$.

Etape 200 : Estimation d'une intensité d'émission correspondant à l'image reconstruite $I^{x}$.

A partir des $W'$ carrés des modules des d'amplitudes complexe $s_{w',\lambda}^{x}(r)$ résultant de l'étape 190, on peut estimer, en chaque voxel objet, une intensité d'émission de fluorescence $S_{I^{x}}^{x}(r)$ correspondant à l'image reconstruite $I^{x}$.

$$S_{I^{x}}^{x}(r) = \frac{1}{W'}\sum_{w'}\left|s_{w',\lambda}^{x}(r)\right|^{2} \quad (19)$$

La matrice de passage $L^{T}$ n'est pas déterminée analytiquement, mais est estimée par les propagations mises en œuvre dans l'étape 190 et dont les résultats sont moyennés lors de l'étape 210. Autrement dit, les étapes 180 à 210 sont équivalentes à la définition de la matrice de passage $L^{T}$. La notation $S_{I^{x}}^{x}(r)$ exprime le fait qu'il s'agit d'une estimation de $S(r)$ à l'itération de rang $x$, effectuée sur la base de l'image reconstruite $I^{x}$.

Si $S_{I^{x}}^{x}$ correspond à un vecteur dont chaque terme est égal à $S_{I^{x}}^{x}(r)$, on a :

$$S_{I^{x}}^{x} \approx L^{T}I^{x} \quad (20)$$

et, sachant que $I^{x} \approx LF^{x}$,

$$S_{I^{x}}^{x} \approx L^{T}LF^{x} \quad (21)$$

Les étapes 210 à 240 décrites ci-dessous sont équivalentes aux étapes 170 à 200. Tandis que dans les étapes 170 à 200, permettent une rétropropagation de l'image reconstruite $I^{x}$, les étapes 210 à 240 effectuent, selon la même approche, une rétropropagation de l'image acquise $I$. Tandis que l'image reconstruite $I^{x}$ résulte d'un algorithme de reconstruction, et correspond donc à une simulation, l'image acquise $I$ correspond aux données mesurées.

Etape 210. Définition d'amplitudes complexes dans chaque voxel image $r'$ de l'image acquise $I$.

Sous-étape 211 : Assignation de valeurs de phase à chaque voxel image de l'image acquise $I$. De façon analogue aux étapes 131 et 171, une valeur de phase aléatoire $\Phi_{r',w''}$ est assignée à chaque voxel image $I(r')$. $w''$ est un rang de répétition.

Sous-étape 212 : Assignation d'une longueur d'onde

De façon analogue aux étapes 132 et 172, on assigne une longueur d'onde d'émission $\lambda$ à chaque voxel image. La longueur d'onde d'émission est assignée en prenant en compte la probabilité d'émission $P^{\lambda}$ dans la bande spectrale d'émission, définie par la distribution de probabilités $P$. La sous-étape 212 est optionnelle.

Les sous-étapes 211 et 212 permettent de définir, en chaque voxel image $r'$, une amplitude complexe $u_{w'',\lambda}^{x}(r')$ telle que :

$$u_{w'',\lambda}^{x}(r') = \sqrt{I(r')}e^{i\Phi_{r',w''}} \quad (22)$$

Etape 220: Rétropropagation

Au cours de cette étape, on applique un algorithme de rétropropagation de la lumière permettant de modéliser une amplitude complexe se propageant à partir du capteur d'image, vers travers chaque voxel objet $r$. Il s'agit de déterminer un ensemble de valeurs d'amplitudes complexe d'émission de fluorescence $s^x_{w,\lambda}(r)$, dans chaque voxel objet, expliquant l'image acquise $I$ résultant de l'étape 120.

L'algorithme de rétropropagation est analogue à celui mise en œuvre dans l'étape 180. Il permet d'estimer $s^x_{w'',\lambda}(r)$, telle que

$$s^x_{w'',\lambda}(r) = \int G^{\lambda H}(r,r') u^x_{w'',\lambda}(r')dr' \quad (23)$$

où $H$ désigne l'opérateur Hilbertien.

Suite à la rétropropagation, on dispose, en chaque voxel objet, d'une amplitude complexe d'émission de fluorescence $s_{w'',\lambda}(r)$ correspondant à l'image acquise $I$.

Etape 230 : répétition des étapes 210 et 220, $W'$ étant un entier désignant le nombre de répétitions. $W''$ est par exemple égal à 1000.

Suite à l'étape 200, on dispose, en chaque voxel objet, de $W''$ valeurs d'amplitudes complexe $s^x_{w'',\lambda}(r)$.

Etape 240 : Estimation d'une intensité d'émission correspondant à l'image acquise.

A partir des $W''$ valeurs d'amplitude complexe $s^x_{w'',\lambda}(r)$ résultant de l'étape 230, on peut estimer, en chaque voxel objet, une intensité d'émission de fluorescence correspondant à l'image acquise $I$. L'intensité d'émission peut être obtenue à partir d'une moyenne des carrés des modules des $W''$ amplitudes complexes $s^x_{w'',\lambda}(r)$ résultant de l'étape 230. Ainsi,

$$S^x_I(r) = \frac{1}{W''} \sum_{w''} \left| s^x_{w'',\lambda}(r) \right|^2 \quad (24)$$

La notation $S^x_I(r)$ exprime le fait qu'il s'agit d'une estimation de $S^x_I(r)$ à l'itération de rang $x$, effectuée sur la base de l'image acquise $I$.

Si $S^x_I$ prend la forme d'un vecteur dont chaque terme est égal à $S^x_I(r)$, on a :

$$S^x_I \approx L^T I \quad (25)$$

Etape 250 : mise à jour

Au cours de l'étape 250, la distribution de fluorescence $F^x$ est mise à jour selon l'expression :

$$F^{x+1} \leftarrow F^x \frac{L^T I}{L^T L F^x} \quad (26)$$

Ainsi, chaque terme $F^{x+1}(r)$ est mis à jour de telle sorte que :

$$F^{x+1}(r) \leftarrow F^x(r) \frac{S^x_I(r)}{S^x_{I^x}(r)} \quad (27)$$

L'opérateur $\leftarrow$ est « est remplacé par ».

L'expression (26) correspond à une mise à jour par un algorithme de type FMN (Factorisation en Matrices non Négatives).

Etape 260 : Réitération des étapes 130 à 250. Le critère d'arrêt d'itérations peut être un nombre d'itérations prédéterminé ou une différence faible entre deux vecteurs successifs $F^x$ avant et après la mise à jour.

Selon une variante, les étapes 170 à 200 consistent non pas à rétropropager l'image reconstruite $I^x$, mais une image différentielle, représentative d'une comparaison entre l'image reconstruite $I^x$ et l'image acquise $I$. Au cours de l'étape 170, on définit une image différentielle $\Delta I^x$ telle que

$$\Delta I^x = \|I - I^x\|^2 \quad (28)$$

Les étapes 171 et 172 sont mises en œuvre de façon similaire à ce qui a été préalablement décrit, de façon à définir, en chaque voxel image $r'$, une amplitude complexe, dite différentielle, $u_{w',\lambda}(r')$ telle que :

$$u_{w',\lambda}(r') = \sqrt{\Delta I^x(r')}\, e^{i\Phi_{r',w'}} \quad (29)$$

L'étape 180 préalablement décrite est mise en œuvre en utilisant, en tant que données d'entrées, les amplitudes complexes différentielles $u_{w',\lambda}(r')$ telles que décrites dans (29). On obtient ainsi une amplitude différentielle $\Delta s^x_{w',\lambda}$, qui correspond à une émission différentielle de fluorescence, en chaque voxel objet, permettant d'expliquer l'image différentielle $\Delta I^x$. L'étape 190 est mise en œuvre, de façon à effectuer une répétition de W' étapes 170 et 180. L'étape 200 est effectuée, en effectuant, pour chaque voxel objet, une moyenne $\Delta \bar{s}^x_\lambda(r)$ des W' carrés des modules des amplitudes différentielle $\Delta s^x_{w',\lambda}(r)$.

On peut ainsi obtenir, en chaque voxel, une intensité différentielle telle que :

$$\Delta S^x(r) = |\Delta \bar{s}^x_\lambda(r)|^2 = \frac{1}{W'}\sum_{w'}\left|\Delta s^x_{w',\lambda}(r)\right|^2 \quad (30)$$

Selon ce mode de réalisation, les étapes 210 à 240 ne sont pas mises en œuvre. Lors de l'étape 250, la formule de mise à jour consiste à définir une valeur de $F^{x+1}$ permettant de diminuer la norme, par exemple la norme L2, du vecteur $\Delta S^x$. L'algorithme d'optimisation peut être un algorithme de type descente de gradient. Il permet de déterminer une distribution de fluorescence $F^x$ qui est utilisée lors d'une réitération des étapes 130 à 200 ainsi que 250.

L'inventeur considère qu'il est préférable qu'une formule de mise à jour de type FMN soit utilisée.

Quel que soit le mode de réalisation, suite à la dernière itération, on obtient une distribution d'intensité de fluorescence, correspondant au vecteur $F^x$ résultant de la dernière itération.

On sait qu'une intensité de fluorescence correspond à un rendement de fluorescence $\eta(r)$ multiplié par une intensité lumineuse d'excitation. L'intensité lumineuse d'excitation $U_{ex}(r)$ correspond à l'intensité de l'onde lumineuse d'illumination atteignant chaque voxel objet au cours de l'étape 110. Si $U_{ex}(r)$ est connue en chaque voxel objet, à partir de chaque terme $F^x(r)$ du vecteur $F^x$, on peut estimer $\eta(r)$ selon l'expression :

$$\eta(r) = \frac{F^x(r)}{U_{ex}(r)} \quad (31)$$

Cela permet d'obtenir une carte de fluorescence $\eta$ dans chaque voxel de l'objet. La carte de fluorescence est indépendante de l'illumination. Elle correspond à une quantité de fluorophore dans chaque voxel objet de l'objet. La carte de fluorescence $\eta$ est un vecteur défini en chaque voxel objet

L'inventeur a mis en œuvre le procédé décrit en lien avec les étapes 100 à 250, en utilisant une formule de mise à jour de type FMN.

Les dimensions de l'objet étaient :

- largeur et longueur selon les axes X et Y : 150 $\mu$m ;
- épaisseur selon Z : 200 $\mu$m ;
- indice optique : 1.33, sauf dans une boule de rayon 30 $\mu$m centrée en (0, 0, -40 $\mu$m), dans lequel l'indice est égal à 1.33 + 0.05, l'origine du repère étant au centre de l'objet ;
- positions des sources de fluorescence : deux sources ponctuelles en (0, 0, -80 $\mu$m) et (0, 30, - 80 $\mu$m) ;
- spectre de fluorescence : distribution gaussienne centrée sur longueur d'onde de 0.505 $\mu$m de largeur spectrale 0.020 $\mu$m ;
- maillage des voxels : $\Delta x = 0.14$ $\mu$m ; $\Delta y = 0.14$ $\mu$m ; $\Delta z = 2$ $\mu$m .

Des images de fluorescence ont été acquises à différentes profondeurs, selon un pas spatial $\Delta z = 2$ $\mu$m: 50

images ont ainsi été acquises selon z = - 100 $\mu$m à z = 0 $\mu$m.

La figure 5A représente la distribution spatiale d'indices de réfraction dans l'objet. Sur la figure 5A, on a représenté les deux sources ponctuelles (s) ainsi que la boule de rayon 30 $\mu$m induisant une variation d'indice $\delta n$

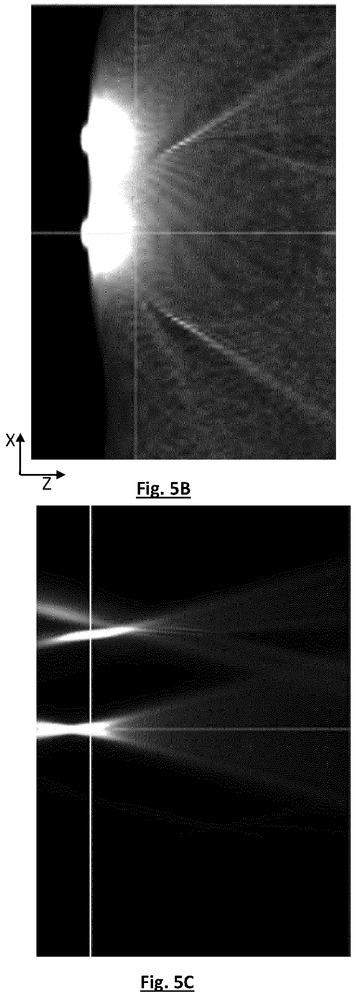

La figure 5B représente l'intensité d'une onde lumineuse de fluorescence se propageant à travers chaque voxel objet. Chaque valeur représentée sur la figure 5B est :

$$U^x(r) = |\bar{u}^x_\lambda(r)|^2 = \frac{1}{W}\sum_w \left|u^x_{w,\lambda}(r)\right|^2 \quad (32)$$

avec $u^x_{w,\lambda}(r) = u^{x,+}_{w,k}(r)$ pour chaque couche $k$ dans l'objet. $u^x_{w,\lambda}(r)$ correspond à l'amplitude d'une onde lumineuse complexe se propageant à travers chaque voxel de l'objet, $u^x_{w,\lambda}(r)$ étant estimé par le modèle de propagation BPM tel que décrit dans l'étape 140. Ainsi, la figure 5B correspond à une coupe, parallèlement au plan XZ, de la mise en œuvre de l'algorithme de propagation.

La figure 5C correspond à une coupe de chaque image de fluorescence acquise, parallèlement à un plan XZ, entre les profondeurs z = - 100 $\mu$m à z = 0 $\mu$m. L'ouverture numérique du système de mesure (capteur + système optique) était de 0.4. On observe que compte tenu de la non uniformité des indices à l'intérieur de l'objet, la mesure de fluorescence est décalé selon l'axe Z. Sur la figure 5C, la bande verticale correspond à la position réelle selon l'axe Z.

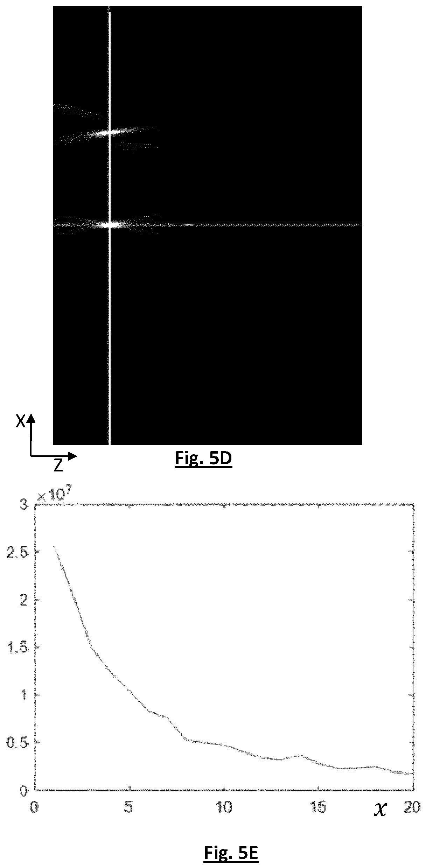

La figure 5D représente une coupe d'une distribution d'émission de fluorescence $F^x$ obtenue par FMN après 20 itérations. On voit que la position des sources en Z est rétablie par rapport à la position indiquée par les images mesurées. Le procédé permet ainsi de tenir compte de la non uniformité de l'indice de réfraction, pour positionner les sources d'émission de lumière de fluorescence.

Au cours de chaque itération, on a calculé un critère d'attache aux données $\varepsilon^x$, correspondant à une différence entre l'image reconstruite ($I^x \approx L\, F^x$) et l'image acquise ($I$).

$$\varepsilon^x = \|I - LF^x\|^2 \quad (33)$$

La figure 5E montre l'évolution du critère $\varepsilon^x$ (axe des ordonnées) en fonction du rang x de chaque itération. (axe des abscisses).

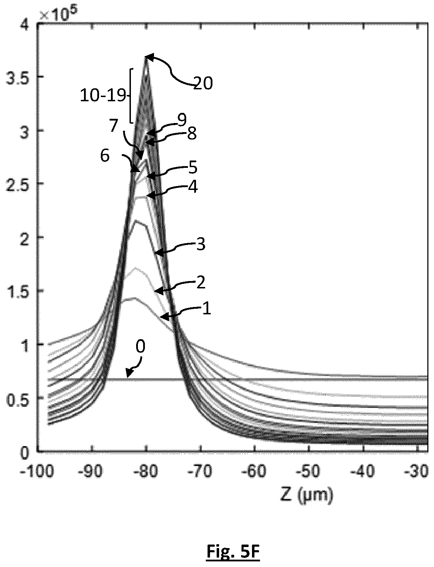

Au cours de chaque itération, on a calculé la somme de l'intensité de l'onde lumineuse dans chaque plan s'étendant parallèlement à l'axe Z, entre - 100 $\mu$m et 0 $\mu$m. Il s'agit de la somme de chaque terme $F^x(r)$ pour les voxels objet s'étendant selon la même profondeur Z. La figure 5F représente un profil des intensités pour chaque couche, au cours de chaque itération. L'axe des abscisses correspond à une coordonnée de chaque couche selon l'axe Z et l'axe des ordonnées montre la somme $F^x(r)$ pour chaque profondeur selon l'axe Z. Lors de l'initialisation, la distribution $F^x$ était homogène, ce qui correspond à la courbe « 0 ». Au fur et à mesure des itérations, l'intégrale se « pique » sur le plan qui comporte les sources de fluorescence. On a indiqué le rang d'itérations sur certains profils. Plus les itérations progressent, plus le profil se pique et tend à se décaler selon l'axe Z.

L'invention pourra être mise en œuvre sur des tissus biologiques, à des fins d'aide au diagnostic. Elle pourra également être mise en œuvre, en microscopie, dans des applications liées à la culture cellulaire ou à la culture de microorganismes, dans des milieux de culture, sous réserve d'une transmittance suffisante à l'égard de la lumière d'illumination et de la lumière de fluorescence. Plus généralement, l'invention peut être appliquée à l'analyse d'objets solides, liquides ou prenant la forme de gels, dans le domaine de l'agroalimentaire ou d'autres domaines industriels.

**Revendications**

1. Procédé de reconstruction d'une distribution spatiale de fluorescence (*F*), tridimensionnelle, à l'intérieur d'un objet (10), l'objet étant susceptible d'émettre une lumière de fluorescence sous l'effet d'une illumination dans une bande spectrale d'excitation, l'objet étant discrétisé en voxels objet (*r*), de telle sorte que :

   - chaque voxel objet est susceptible d'émettre une lumière dans une bande spectrale de fluorescence ;
   - la distribution spatiale de fluorescence définit une intensité d'émission de lumière de fluorescence dans chaque voxel objet;

   le procédé comportant les étapes suivantes ,

- a) disposition de l'objet face à un capteur d'image (15), le capteur d'image étant configuré pour former une image dans un plan focal objet, le plan focal objet étant configuré pour s'étendre successivement en différentes profondeur dans l'objet ($d_1....d_n...d_N$) ;
- b) illumination de l'objet dans la bande spectrale d'excitation, et acquisition successive de plusieurs images élémentaires ($I_1....I_n...I_N$), le plan focal objet s'étendant respectivement aux différentes profondeurs de l'objet, chaque image étant définie selon des pixels, à chaque pixel correspondant une valeur d'intensité mesurée, les images élémentaires formant une image acquise ($I$) tridimensionnelle, chaque pixel d'une image élémentaire formant un voxel image de l'image acquise ;
- c) initialisation de la distribution spatiale de fluorescence ;

le procédé étant **caractérisé en ce qu'**il comporte également :

- d) prise en compte de la distribution de fluorescence initiale ($F^0$, $F^x$) ou résultant d'une itération précédente ;
- e) assignation d'une valeur aléatoire de phase ($\Phi_{r,w}$) à chaque voxel objet, et mise en œuvre d'un algorithme de propagation de la lumière à travers l'objet, de façon à estimer une amplitude complexe de l'onde lumineuse détectée par chaque voxel image ( $u_{w,\lambda}^x(r')$ ), l'algorithme de propagation de la lumière prenant en compte une variation de l'indice de réfraction entre deux voxels objet adjacents ;
- f) répétition de W fois de l'étape e), W étant un entier positif, de façon à obtenir, en chaque voxel image, W estimations d'amplitudes complexes ( $u_{w,\lambda}^x(r')$ );
- g) en chaque voxel image, à partir des W estimations résultant de l'étape f), calcul d'une intensité reconstruite ($I^x(r')$), l'ensemble des intensités reconstruites pour chaque voxel image formant une image reconstruite ($I^x$) ;
- h) formation éventuelle d'une image différentielle ($\Delta I^x$), l'image différentielle correspondant à une comparaison entre l'image reconstruite ($I^x$) et l'image acquise ($I$) ;
- i) assignation d'une valeur aléatoire de phase ($\Phi_{r',w'}$) à chaque voxel image de l'image acquise résultant de l'étape g) ou de l'image différentielle résultant de l'étape h), et mise en œuvre d'un algorithme de rétropropagation de la lumière à travers l'objet, de façon à obtenir, dans chaque voxel objet :

   • une amplitude complexe d'émission de fluorescence correspondant à l'image reconstruite résultant de l'étape g) ;
   • ou une amplitude complexe différentielle d'émission de fluorescence correspondant à l'image différentielle résultant de l'étape h) ;

- j) répétition de W' fois de l'étape i), W' étant un entier positif, de façon à obtenir, pour chaque voxel objet, W' estimations d'amplitudes complexes ( $s_{w,\lambda}^x(r)$ ) correspondant à l'image reconstruite ou W' estimations d'amplitudes complexes différentielles ( $\Delta s_{w',\lambda}^x$ ) correspondant à l'image différentielle ;
- k) à partir des W' estimations résultant de l'étape j), calcul, en chaque voxel objet, d'une intensité d'émission ( $S_{I^x}^x(r)$ ) ou d'une intensité d'émission différentielle ($\Delta S^x(r)$) ;
- l) mise à jour de la distribution spatiale de fluorescence dans l'objet ($F^{x+1}$) à partir de l'intensité d'émission ( $S_{I^x}^x$ ) ou de l'intensité d'émission différentielle ($\Delta S^x$) calculée dans chaque voxel de l'objet lors de l'étape k) ;
- m) réitération des étapes d) à l) jusqu'à l'atteinte d'un critère d'arrêt des itérations.

2.  Procédé selon la revendication 1, dans lequel le procédé comportant, à chaque itération, les étapes suivantes :

   • i-bis) assignation d'une valeur aléatoire de phase ($\Phi_{r',w'}$) à chaque voxel image de l'image acquise lors de b) et mise en œuvre d'un algorithme de réropropagation de la lumière à travers l'objet, de façon à estimer, dans chaque voxel objet, une amplitude complexe d'émission de fluorescence correspondant à l'image acquise ( $s_{w'',\lambda}^x(r)$ );
   • j-bis) répétition de W'' fois de l'étape i-bis), W'' étant un entier positif, de façon à obtenir, pour chaque voxel objet, W'' estimations d'amplitudes complexe d'émission de fluorescence correspondant à l'image acquise ;
   • k-bis) à partir des W'' estimations résultant de l'étape j-bis), calcul d'une intensité d'émission, en chaque voxel objet, correspondant à l'image acquise ( $S_I^x(r)$ ) ;

le procédé étant tel que lors de l'étape l), la mise à jour est effectuée à partir d'un ratio ( $\frac{S_I^x(r)}{S_{I^x}^x(r)}$ ), calculé pour chaque voxel objet, entre l'intensité d'émission correspondant à l'image acquise et l'intensité d'émission correspondant à l'image reconstruite.

3. Procédé selon la revendication 2, dans lequel lors de chaque répétition de l'étape i-bis), la valeur de phase assignée à chaque voxel image de l'image acquise ($I$) correspond à la valeur de phase assignée au même voxel image de l'image reconstruite ($I^x$) lors d'une étape i) de la même itération.

4. Procédé selon la revendication 2 ou la revendication 3, dans lequel lors de chaque étape k-bis), pour chaque voxel objet, l'intensité d'émission est calculée à partir d'une moyenne des W" intensités d'émission estimées, pour ledit voxel objet, lors de chaque étape i-bis).

5. Procédé selon la revendication 1, dans lequel :

- lors de l'étape h), l'image différentielle ($\Delta I^x$) est une différence quadratique entre l'image reconstruite ($I^x$) et l'image acquise ($I$) ;
- chaque étape i) comporte une obtention, dans chaque voxel image, d'une amplitude complexe différentielle, calculée à partir de l'image différentielle ;
- l'étape k) comporte un calcul d'une intensité d'émission différentielle, en chaque voxel objet, correspondant à l'image différentielle calculée en h) ;
- lors de l'étape l), la mise à jour de la distribution spatiale de fluorescence dans l'objet est effectuée de manière à minimiser l'intensité d'émission différentielle des voxels objets.

6. Procédé selon l'une quelconque des revendications précédentes, dans lequel

- lors de chaque étape g), pour chaque voxel image, l'intensité reconstruite est calculée à partir d'une moyenne du carré des modules des W estimations d'amplitudes complexes, pour ledit voxel image, lors de chaque étape e) ;
- lors de chaque étape k), pour chaque voxel objet, l'intensité d'émission est calculée à partir d'une moyenne du carré des modules des W' estimations amplitudes complexes d'émission de fluorescence, pour ledit voxel, lors de chaque étape i).

7. Procédé selon l'une quelconque des revendications précédentes, dans lequel

- lors de chaque étape e), une longueur d'onde est assignée à chaque voxel objet, dans la bande spectrale de fluorescence, la longueur d'onde étant assignée de façon aléatoire ou selon une distribution de probabilité prédéterminée ;
- lors de chaque étape i), une longueur d'onde est assignée à chaque voxel image, dans la bande spectrale de fluorescence, la longueur d'onde étant assignée de façon aléatoire ou selon une distribution de probabilité prédéterminée.

8. Procédé selon l'une quelconque des revendications précédentes, dans lequel lors de l'étape e) et lors de l'étape i), l'algorithme de propagation de la lumière met en œuvre un algorithme de propagation unidirectionnel, de type méthode de propagation de faisceau, de façon à déterminer une lumière se propageant à travers un voxel à partir de la lumière se propageant à partir d'un voxel adjacent.

9. Procédé selon la revendication 8, dans lequel l'indice de réfraction de l'objet varie entre deux voxels objet différents.

10. Dispositif d'observation d'un objet, le dispositif comportant :

- un capteur d'image (15), configuré pour former une image dans un plan objet, la distance entre le capteur d'image et le plan objet étant variable ;
- une source de lumière (11), configurée pour illuminer un objet ;
- une unité de traitement (20), configurée pour recevoir des images acquises par le capteur d'image, et mettre en œuvre les étapes c) à l) d'un procédé selon l'une quelconque des revendications précédentes.

11. Support d'enregistrement, lisible par un ordinateur ou pouvant être connecté à un ordinateur, le support d'enregistrement étant configuré pour mettre en œuvre les étapes c) à m) d'un procédé selon l'une quelconque des revendications 1 à 9 à partir d'images formées par un capteur d'image en différentes profondeurs dans un objet.

**Patentansprüche**

1. Verfahren zur Rekonstruktion einer dreidimensionalen räumlichen Fluoreszenzverteilung ($F$), innerhalb eines Objekts (10), wobei das Objekt unter Einwirkung einer Beleuchtung in einem Anregungsspektralband Fluoreszenzlicht emittieren kann, wobei das Objekt in Objektvoxeln ($r$) diskretisiert ist, so dass:

   - jeder Objektvoxel Licht in einem Fluoreszenzspektralband emittieren kann;
   - die räumliche Fluoreszenzverteilung eine Fluoreszenzlichtemissionsintensität in jedem Objektvoxel definiert; das Verfahren umfassend die folgenden Schritte:

      - a) Anordnung des Objekts gegenüber einem Bildsensor (15), wobei der Bildsensor so konfiguriert ist, dass er ein Bild in einer Objektbrennebene erzeugt, wobei die Objektbrennebene so konfiguriert ist, dass sie sich nacheinander in verschiedene Tiefen in das Objekt erstreckt ($d_1....d_n....d_N$) ;
      - b) Beleuchtung des Objekts im Anregungsspektralbereich und aufeinanderfolgende Aufnahme mehrerer Elementarbilder ($I_1....I_n....I_N$), wobei sich die Objektbrennebene jeweils in unterschiedlichen Tiefen des Objekts erstreckt, jedes Bild durch Pixel definiert ist, jedem Pixel ein gemessener Intensitätswert zugeordnet ist und die Elementarbilder ein dreidimensionales aufgenommenes Bild ($I$) erzeugen, wobei jedes Pixel eines Elementarbilds einen Bildvoxel des aufgenommenen Bilds erzeugt;
      - c) Initialisierung der räumlichen Fluoreszenzverteilung;

   wobei das Verfahren **dadurch gekennzeichnet ist, dass** es außerdem umfasst:

      - d) Berücksichtigung der anfänglichen oder aus einer vorherigen Iteration ($F^0$, $F^x$) resultierenden Fluoreszenzverteilung;
      - e) Zuweisung eines zufälligen Phasenwertes ($\Phi_{r,w}$) zu jedem Objektvoxel, und Ausführung eines Algorithmus zur Ausbreitung des Lichts durch das Objekt, um eine komplexe Amplitude der von jedem Bildvoxel ( $u_{w,\lambda}^x(r')$ ) erfassten Lichtwelle zu schätzen, wobei der Algorithmus zur Ausbreitung des Lichts eine Änderung des Brechungsindexes zwischen zwei benachbarten Objektvoxeln berücksichtigt;
      - f) Wiederholung des Schritts e) W-mal, wobei W eine positive ganze Zahl ist, um in jedem Bildvoxel W Schätzungen komplexer Amplituden zu erhalten ( $u_{w,\lambda}^x(r')$ ) ;
      - g) in jedem Bildvoxel Berechnung einer rekonstruierten Intensität ($I^x(r')$) aus den W Schätzungen, die sich aus Schritt f) ergeben, wobei die Gesamtheit der rekonstruierten Intensitäten für jeden Bildvoxel ein rekonstruiertes Bild erzeugt ($I^x$);
      - h) gegebenenfalls Erzeugung eines differentiellen Bilds ($\Delta I^x$), wobei das differentielle Bild einem Vergleich zwischen dem rekonstruierten Bild ($I^x$) und dem aufgenommenen Bild entspricht ($I$);
      - i) Zuweisung eines zufälligen Phasenwertes ($\Phi_{r',w'}$) zu jedem Bildvoxel des aufgenommenen Bilds, der sich aus Schritt g) ergibt oder dem aus Schritt h) resultierenden differentiellen Bild, und Ausführung eines Algorithmus zur Rückwärtsausbreitung des Lichts durch das Objekt, um in jedem Objektvoxel Folgendes zu erhalten:

         • eine komplexe Fluoreszenzemissionsamplitude, die dem aus Schritt g) resultierenden rekonstruierten Bild entspricht;
         • oder eine komplexe differentielle Fluoreszenzemissionsamplitude, die dem aus Schritt h) resultierenden differentiellen Bild entspricht;

      - j) Wiederholen von Schritt i) W'-mal, wobei W' eine positive ganze Zahl ist, um für jeden Objektvoxel W' Schätzungen komplexer Amplituden ( $s_{w,\lambda}^x(r)$ ), die dem rekonstruierten Bild entsprechen, oder W' Schätzungen komplexer differentieller Amplituden ( $\Delta s_{w',\lambda}^x$ ), die dem differentiellen Bild entsprechen, zu erhalten;
      - k) aus den W' Schätzungen, die sich aus Schritt j) ergeben, Berechnung einer Emissionsintensität (

$S_{I^x}^x(r)$ ) oder einer differentiellen Emissionsintensität ($\Delta S^x(r)$) in jedem Objektvoxel;

- l) Aktualisierung der räumlichen Fluoreszenzverteilung in dem Objekt ($F^{x+1}$) anhand der Emissionsintensität ( $S_{I^x}^x$ ) oder der differentiellen Emissionsintensität ($\Delta S^x$), die in jedem Voxel des Objekts in Schritt k) berechnet wurde;
- m) Wiederholung der Schritte d) bis l), bis ein Kriterium für das Beenden der Iterationen erreicht ist.

2. Verfahren nach Anspruch 1, wobei das Verfahren bei jeder Iteration die folgenden Schritte umfasst:

- i-II) Zuweisung eines zufälligen Phasenwerts ($\Phi_{r',w''}$) zu jedem Bildvoxel des in Schritt b) aufgenommenen Bilds und Ausführung eines Algorithmus zur Rückwärtsausbreitung des Lichts durch das Objekt, um in jedem Objektvoxel eine komplexe Fluoreszenzemissionsamplitude zu schätzen, die dem aufgenommenen Bild ( $S_{w'',\lambda}^x(r)$ ) entspricht;
- j-II) Wiederholen des Schritts i-II) W''-mal, wobei W'' eine positive ganze Zahl ist, um für jeden Objektvoxel W'' Schätzungen der komplexen Fluoreszenzemissionsamplituden zu erhalten, die dem aufgenommenen Bild entsprechen;
- k-II) anhand der W'' Schätzungen, die sich aus Schritt j-II) ergeben, Berechnung einer Emissionsintensität in jedem Objektvoxel, die dem aufgenommenen Bild ( $S_I^x(r)$ ) entspricht;

wobei das Verfahren so beschaffen ist, dass in Schritt l) die Aktualisierung anhand eines für jeden Objektvoxel berechneten Verhältnisses ( $\frac{S_I^x(r)}{S_{I^x}^x(r)}$ ) zwischen der Emissionsintensität, die dem aufgenommenen Bild entspricht, und der Emissionsintensität, die dem rekonstruierten Bild entspricht, erfolgt.

3. Verfahren nach Anspruch 2, wobei bei jeder Wiederholung des Schritts i-II) der jedem Bildvoxel des aufgenommenen Bilds ($I$) zugewiesene Phasenwert dem Phasenwert entspricht, der dem gleichen Bildvoxel des rekonstruierten Bilds ($I^x$) in einem Schritt i) derselben Iteration zugewiesen wurde.

4. Verfahren nach Anspruch 2 oder Anspruch 3, wobei bei jedem Schritt k-II) für jedes Objektvoxel die Emissionsintensität aus einem Mittelwert der Emissionsintensitäten W'' berechnet wird, die für das Objektvoxel in jedem Schritt i-II) geschätzt wurden.

5. Verfahren nach Anspruch 1, wobei:

- in Schritt h) das differentielle Bild ($\Delta I^x$) eine quadratische Differenz zwischen dem rekonstruierten Bild ($I^x$) und dem aufgenommenen Bild ($I$) ist;
- jeder Schritt i) das Erhalten einer komplexen differentiellen Amplitude in jedem Bildvoxel umfasst, die anhand des differentiellen Bilds berechnet wird;
- Schritt k) eine Berechnung einer differentiellen Emissionsintensität in jedem Objektvoxel umfasst, die dem in h) berechneten differentiellen Bild entspricht;
- in Schritt l) die räumliche Verteilung der Fluoreszenz in dem Objekt so aktualisiert wird, dass die differentielle Emissionsintensität der Objektvoxels minimiert wird.

6. Verfahren nach einem der vorhergehenden Ansprüche, wobei:

- in jedem Schritt g) für jeden Bildvoxel die rekonstruierte Intensität aus einem Mittelwert des Quadrats der Module der W Schätzungen der komplexen Amplituden für den Bildvoxel in jedem Schritt e) berechnet wird;
- in jedem Schritt k) für jeden Objektvoxel die Emissionsintensität aus einem Mittelwert des Quadrats der Module der W' Schätzungen der komplexen Fluoreszenzemissionsamplituden für den Voxel in jedem Schritt i) berechnet wird.

7. Verfahren nach einem der vorhergehenden Ansprüche, wobei:

- in jedem Schritt e) jedem Objektvoxel eine Wellenlänge in dem Fluoreszenzspektralband zugewiesen wird, wobei die Wellenlänge zufällig oder gemäß einer vorbestimmten Wahrscheinlichkeitsverteilung zugewiesen wird;
- in jedem Schritt i) jedem Bildvoxel eine Wellenlänge in dem Fluoreszenzspektralband zugewiesen wird, wobei die Wellenlänge zufällig oder gemäß einer vorbestimmten Wahrscheinlichkeitsverteilung zugewiesen wird.

8. Verfahren nach einem der vorhergehenden Ansprüche, wobei in Schritt e) und in Schritt i) der Lichtausbreitungsalgorithmus einen unidirektionalen Ausbreitungsalgorithmus vom Typ einer Strahlausbreitungsmethode ausführt, um ein sich durch einen Voxel ausbreitendes Licht aus dem sich von einem benachbarten Voxel ausbreitenden Licht zu bestimmen.

9. Verfahren nach Anspruch 8, wobei der Brechungsindex des Objekts zwischen zwei verschiedenen Objektvoxeln variiert.

10. Vorrichtung zum Beobachten eines Objekts, die Vorrichtung umfassend:

- einen Bildsensor (15), der so konfiguriert ist, dass er ein Bild in einer Objektebene erzeugt, wobei der Abstand zwischen dem Bildsensor und der Objektebene variabel ist;
- eine Lichtquelle (11), die so konfiguriert ist, dass sie ein Objekt beleuchtet;
- eine Verarbeitungseinheit (20), die so konfiguriert ist, dass sie von dem Bildsensor aufgenommene Bilder empfängt und die Schritte c) bis l) eines Verfahrens nach einem der vorhergehenden Ansprüche ausführt.

11. Aufzeichnungsmedium, das von einem Computer lesbar oder mit einem Computer verbindbar ist, wobei das Aufzeichnungsmedium so konfiguriert ist, dass es die Schritte c) bis m) eines Verfahrens nach einem der Ansprüche 1 bis 9 anhand von Bildern ausführt, die von einem Bildsensor in unterschiedlichen Tiefen in einem Objekt erzeugt wurden.

## Claims

1. Method for reconstructing a three-dimensional spatial distribution of fluorescence ($F$) inside an object (10), the object being capable of emitting fluorescence light under the effect of illumination in an excitation spectral band, the object being discretized into object voxels ($r$), such that:

- each object voxel is capable of emitting light in a fluorescence spectral band;
- the fluorescence spatial distribution defines an intensity of emission of fluorescence light in each object voxel;

the method comprising the following steps:

- a) placing the object facing an image sensor (15), the image sensor being configured to form an image in an object focal plane, the object focal plane being configured to lie successively at various depths in the object ($d_1....d_n...d_N$);
- b) illuminating the object in the excitation spectral band, and successively acquiring a plurality of elementary images ($I_1....I_n...I_N$), the object focal plane respectively lying at the various depths in the object, each image being divided into pixels, to each pixel corresponding one measured intensity value, the elementary images forming a three-dimensional acquired image ($I$), each pixel of an elementary image forming one image voxel of the acquired image;
- c) initializing the fluorescence spatial distribution;

the method being **characterized in that** it also comprises :

- d) taking into account the initial fluorescence distribution ($F^0$, $F^x$) or the fluorescence distribution resulting from a preceding iteration;
- e) assigning a random phase value ($\Phi_{r,w}$) to each object voxel, and implementing an algorithm modelling propagation of light through the object, so as to estimate a complex amplitude of the light wave detected by each image voxel ( $u_{w,\lambda}^x(r')$ ), the algorithm modelling propagation of light accounting for a variation in refractive index between two adjacent object voxels.;
- f) repeating step e) W times, W being a positive integer, so as to obtain, for each image voxel, W complex-amplitude estimates ( $u_{w,\lambda}^{x}(r')$ );

- g) for each image voxel, on the basis of the W estimates resulting from step f), computing a reconstructed intensity ($I^x(r')$), all of the reconstructed intensities of each image voxel together forming a reconstructed image ($I^x$) ;

- h) optionally forming a differential image ($\Delta I^x$), the differential image corresponding to a comparison between the reconstructed image ($I^x$) and the acquired image ($I$);

- i) assigning a random phase value ($\Phi_{r',w'}$) to each image voxel of the acquired image resulting from step g) or of the differential image resulting from step h), and implementing an algorithm modelling back-propagation of light through the object, so as to obtain, for each object voxel:

> • a complex fluorescence-emission amplitude corresponding to the reconstructed image resulting from step g);
> • or a complex differential fluorescence-emission amplitude corresponding to the differential image resulting from step h);

- j) repeating step i) W' times, W' being a positive integer, so as to obtain, for each object voxel, W' complex-amplitude estimates ( $s_{w,\lambda}^{x}(r)$ ) corresponding to the reconstructed image or W' differential complex-amplitude estimates ( $\Delta s_{w',\lambda}^{x}$ ) corresponding to the differential image;

- k) on the basis of the W' estimates resulting from step j), computing, for each object voxel, an emission intensity ( $S_{I^x}^{x}(r)$ ) or a differential emission intensity ($\Delta S^x(r)$);

- l) updating the spatial distribution of fluorescence in the object ($F^{x+1}$) on the basis of the emission intensity ( $S_{I^x}^{x}$ ) or of the differential emission intensity ($\Delta S^x$) computed for each voxel of the object in step k);

- m) reiterating steps d) to l) until a criterion of stoppage of the iterations is met.

2.  Method according to Claim 1, wherein the method comprises, in each iteration, the following steps:

> • i-bis) assigning a random phase value ($\Phi_{r',w''}$) to each image voxel of the image acquired in step b), and implementing an algorithm modelling back-propagation of light through the object, so as to estimate, for each object voxel, a complex fluorescence-emission amplitude corresponding to the acquired image ( $s_{w'',\lambda}^{x}(r)$ );
> • j-bis) repeating step i-bis) W" times, W" being a positive integer, so as to obtain, for each object voxel, W" estimates of complex fluorescence-emission amplitudes corresponding to the acquired image ( $S_{I}^{x}(r)$ );
> • k-bis) on the basis of the W" estimates resulting from step j-bis), computing an emission intensity, for each object voxel, corresponding to the acquired image;

the method being such that, in step l), the update is carried out on the basis of a ratio ( $\dfrac{S_{I}^{x}(r)}{S_{I^x}^{x}(r)}$ ), computed for each object voxel, between the emission intensity corresponding to the acquired image and the emission intensity corresponding to the reconstructed image.

3.  Method according to Claim 2, wherein, in each repetition of step i-bis), the phase value assigned to each image voxel of the acquired image ($I$) corresponds to the phase value assigned to the same image voxel of the image reconstructed ($I^x$) in a step i) of the same iteration.

4.  Method according to Claim 2 or to Claim 3, wherein, in each step k-bis), for each object voxel, the emission intensity is computed on the basis of a mean square of the moduli of the W" fluorescence emission complex amplitudes estimated, for said object voxel, in each step i-bis).

5.  Method according to Claim 1, wherein:

> - in step h), the differential image ($\Delta I^x$) is a quadratic difference between the reconstructed image ($I^x$) and the acquired image ($I$) ;
> - each step i) comprises obtaining, for each image voxel, a differential complex amplitude computed on the basis of the differential image;

- step k) comprises computing a differential emission intensity, for each object voxel, corresponding to the differential image computed in step h);
- in step l), the spatial distribution of fluorescence in the object is updated so as to minimize the differential emission intensity of the object voxels.

6. Method according to any one of the preceding Claims, wherein:

- in each step g), for each image voxel, the reconstructed intensity is computed on the basis of a mean of the square of the moduli of the W complex-amplitude estimates obtained, for said image voxel, in each step e);
- in each step k), for each object voxel, the emission intensity is computed on the basis of a mean of the square of the moduli of the W' estimates of complex fluorescence-emission amplitude obtained, for said image voxel, in each step j).

7. Method according to any one of the preceding Claims, wherein:

- in each step e), a wavelength is assigned to each object voxel, in the fluorescence spectral band, the wavelength being assigned randomly or according to a predetermined probability distribution;
- in each step i), a wavelength is assigned to each image voxel, in the fluorescence spectral band, the wavelength being assigned randomly or according to a predetermined probability distribution.

8. Method according to any one of the preceding Claims, wherein, in step e) and in step i), the algorithm modelling propagation of light employs a one-way propagation model, of BPM type, BPM standing for beam propagation method, so as to determine light propagating through a voxel on the basis of light propagating from an adjacent voxel.

9. Method according to any one of the preceding Claims, wherein the refractive index of the object varies between two different object voxels;

10. Device for observing an object, the device comprising:

- an image sensor (15), configured to form an image in an object plane, the distance between the image sensor and the object plane being variable;
- a light source (11), configured to illuminate an object;
- a processing unit (20), configured to receive images acquired by the image sensor, and to implement steps c) to l) of a method according to any one of the preceding claims.

11. Storage medium able to be read by a computer or able to be connected to a computer, the storage medium being configured to implement steps c) to m) of a method according to any one of claims 1 to 9 on the basis of images formed by an image sensor at various depths in an object.

**Fig. 1A**

**Fig. 1B**

**Fig. 2**

$I_N$

$I_n$

$I$

$I_1$

16

$\Delta$

$10_s$

10

$d_1$

$d_n$

Z

Y

$d_N$

**Fig. 3**

**Fig. 4**

**Fig. 5A**

**Fig. 5B**

**Fig. 5C**

Fig. 5D

Fig. 5E

**Fig. 5F**

**EP 4 205 641 B1**

RÉFÉRENCES CITÉES DANS LA DESCRIPTION

**Documents brevets cités dans la description**

- WO 2011038006 A